# EUROPEAN PATENT APPLICATION

(11) **EP 2 476 450 A1**
(43) Date of publication of application: **18.07.2012**
(21) Application number: 11179222.2
(22) Date of filing: 29.08.2011
(51) Int. Cl.: A61M 5/32

(54) **A pen needle container and a method for bending a needle cannula**

(71) Applicant: NOVO NORDISK A/S, 2880 Bagsvaerd (DK)
(72) Inventor: The designation of the inventor has not yet been filed

(57) **Abstract**

The invention relates to pen needle container (4, 10, 20) for holding and supporting a pen needle assembly (11, 21) and a method for bending the front needle part (15) and/or the back needle part (14) of a pen needle assembly (11, 21). The pen needle assembly (11, 21) comprises a needle cannula (6) secured in a hub (3) such that a front needle part or patient needle part (15) of the needle cannula (6) extend in a distal direction from the hub (3) and a back needle part or cartridge part (14) of the needle cannula (6) extend in a proximal direction from the hub (3). The needle container (4, 10, 20) has a proximal open end (12, 22) and a distal closed end (13, 23). The distal closed end (13, 23) is shaped to facilitate bending of the front needle part (15) and/or the back needle part (14) of the needle assembly (11, 21).

## Description

### THE TECHNICAL FIELD OF THE INVENTION:

The invention relates to a pen needle container configured to immobilize the front needle part and/or the back needle part of a needle cannula of a pen needle assembly and a method for immobilizing such back or front needle part through bending.

### DESCRIPTION OF RELATED ART:

People suffering from diabetes have to inject themselves with insulin at a daily basis. For this purpose a great number of different pen systems have been developed over the last 30 years. The typical diabetes patient will require injections of insulin several times during the course of a day. In order to facilitate such injections the patient will need a number of injection needles and in order to prevent skin infections it is recommended to use a new sterile needle for each new injection.

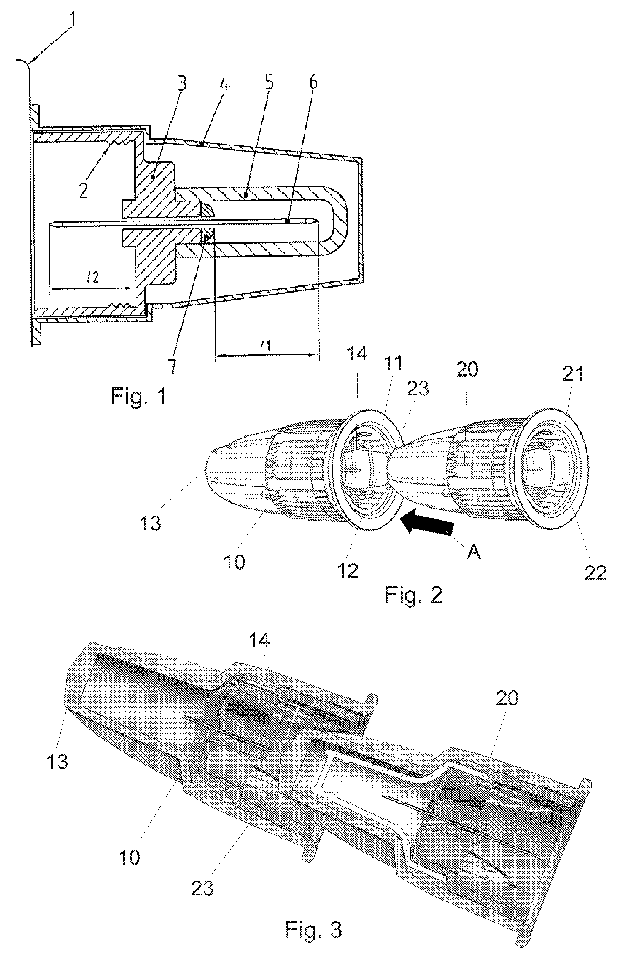

A prior art pen needle unit for an insulin pen system is disclosed in figure 1 and comprises a needle cannula which is mounted in a hub. The needle cannula has an injection part which enters the body during injection and a cartridge part which enters the cartridge contained in the injection pen when the pen needle assembly is connected to the pen system. Both ends of the needle cannula are usually sharp to facilitate the penetration performed by the respective end. The pen needle assembly is usually delivered to the user ready-to-use in a sterile container which has an open proximal end sealed by a protective sheet being impermeable to germs and the like. Further the patient end of the needle cannula is often covered by an inner cap as depictured. The hub usually has connecting means located on its inside making it possible to connect the hub to the pen system. Injection needles for pen injectors are further defined in ISO 11608.

Once the user has performed an injection, the used pen needle assembly should be disposed of. This is often done by returning the used pen needle assembly to its container and disposing it. However it has shown to be beneficial to bend the needle cannula prior to disposing it, thereby minimizing the potential risk that people can obtain physically contact with the needle cannula and thereby be accidentally infected.

US 4,634,428 disclose a cap for a syringe needle made from two telescopic parts which is shaped to bend the tip of an injection needle when one of the parts is telescoped over the tip.

However, this known solution requires an extra element such as a shield. Thus, there is a need for immobilizing either the front needle part and/or the back needle part of a needle cannula without applying extra parts and thereby extra cost to the construction of the needle unit.

### DESCRIPTION OF THE INVENTION:

It is an object of the present invention to provide a pen needle container shaped such that it can facilitate bending of a needle cannula and a method for bending the front needle part and/or the back needle part of a pen needle assembly which can be easily implemented without adding to the cost of producing pen needle units.

The invention is defined in claim 1.

Accordingly the pen needle container holds and supports a pen needle assembly of the type having a needle cannula secured to a hub e.g. by gluing. The needle cannula has a front needle part for entering into the patient, which extend in a distal direction away from the base of the hub and a back needle part for entering into the cartridge of the insulin pen system. The back needle part extends in a proximal direction away from the base of the hub, but is surrounded by the sleeve of the hub. This sleeve usually carries means for attaching the pen needle assembly to the insulin pen system. The pen needle container has a proximal open end and an opposite distal closed end. The distal closed end of the needle container is physically shaped e.g. through moulding such that it can be pressed against the tip of the front needle part and/or the back needle part of the needle cannula of the pen needle assembly in order to bend the part against which it is pressed. The proximal open end has a size that allow the distal closed end of an identical needle container to enter into the open proximal end and obtain contact with the back needle part of the pen needle assembly stored in the pen needle container.

This configuration can be embodied in a multitude of ways. A preferred way would be to shape the distal closed end of the pen needle container into a conical or sphere shape. Alternatively the closed distal end could be provided with a bending protrusion facilitating the bending or it could be provided with a moulded track for guiding the front and/or back needle part during bending. The length that the distal closed end should be able to penetrate into the proximal open end should be sufficient for bending the back needle part.

An other way would be to mould the distal closed end from a material such as a polymer which has a higher hardness than the remaining part of the pen needle container such that the back needle part of the needle cannula deflects and not just sticks into the distal closed end of the pen needle container. This could also be accomplished by adding a coating to the outer surface of the distal closed end, or by moulding an insert of a different material into the distal closed end.

In one example of the invention, the pen needle container can be provided with means locking the pen needle container to the pen needle assembly. These means can e.g. be provided at the closed distal end of the pen needle container. In this manner, the front end of the needle cannula can be fully shielded when the closed distal end of the pen needle container is telescoped over the front part of the needle cannula. The locking means preferably comprises one or more arms provided at the distal end of the pen needle container, which arms are provided with one or more protrusions which interacts with opening or similar means provided on the hub of the pen needle assembly to lock the pen needle container to the pen needle assembly.

The invention also includes a method for bending the back needle of a pen needle assembly as defined in claim 9.

This method comprises the steps of:
(i) Providing a first pen needle container containing a used pen needle assembly,
(ii) Providing a second pen needle container,
(iii) Entering the closed distal end of the second pen needle container into the proximal open end of the first pen needle container,
(iiii) Pressing the first pen needle container and the second pen needle container towards each other.

When the two pen needle containers are pressed together, the distal closed end of one pen needle container will engage and deflect the back needle part of the used needle cannula stored in the other pen needle container. The deflected back needle part will no longer compromise the safety of people handling the disposed needle unit.

Further, the invention includes a method for bending the front needle part of a pen needle assembly. This method is preferably carried out with the pen needle assembly mounted on the injection device i.e. before moving the pen needle assembly into the pen needle container. This method is defined in claim 10 and comprises the steps of:
(i) Providing a pen needle assembly,
(ii) Providing a pen needle container
(iii) Pressing the closed distal end of the pen needle container and the front needle part against each other.

In this way, the pen needle container can be used to immobilize the front part of the needle cannula of the pen needle assembly thereby rendering the pen needle assembly safer when discharged.

### DEFINITIONS:

An "injection pen" is typically an injection apparatus having an oblong or elongated shape somewhat like a pen for writing. Although such pens usually have a tubular cross-section, they could easily have a different cross-section such as triangular, rectangular or square or any variation around these geometries.

As used herein, the term "drug" is meant to encompass any drug-containing flowable medicine capable of being passed through a delivery means such as a hollow needle in a controlled manner, such as a liquid, solution, gel or fine suspension. Representative drugs includes pharmaceuticals such as peptides, proteins (e.g. insulin, insulin analogues and C-peptide), and hormones, biologically derived or active agents, hormonal and gene based agents, nutritional formulas and other substances in both solid (dispensed) or liquid form.

The term "Needle Cannula" is used to describe the actual conduit performing the penetration of the skin during injection. A needle cannula is usually made from a metallic material and connected to a hub to form a complete injection needle also often referred to as a "needle assembly". A needle cannula could however also be made from a polymeric material or a glass material. The needle cannula is mounted in a "hub", which also carries the connecting means for connecting the injection needle to an injection apparatus and is usually moulded from a suitable thermoplastic material. The "connection means" could as examples be a luer coupling, a bayonet coupling, a threaded connection or any combination thereof e.g. a combination as described in EP 1,536,854.

The term "Needle unit" is often used to describe the finished unit as delivered to a costumer, such unit as disclosed in figure 1 consist of a sealed container housing one single needle assembly. Such needle unit or in fact the needle container of a unit usually has a closed distal end and an open proximal end which is sealed by a removable seal. The interior of such container is usually sterile such that the needle assembly is ready-to-use. Needle units special designed for pen injections systems are defined in ISO standard No. 11608 and is often referred to as "pen needles".

"Cartridge" is the term used to describe the container containing the drug. Cartridges are usually made from glass but could also be moulded from any suitable polymer. A cartridge or ampoule is preferably sealed at one end by a pierceable membrane which can be pierced e.g. by the non-patient end of a needle cannula. The opposite end is typically closed by a plunger or piston made from rubber or a suitable polymer. The plunger or piston can be slidable moved inside the cartridge. The space between the pierceable membrane and the movable plunger holds the drug which is pressed out as the plunger decreased the volume of the space holding the drug. However, any kind of container- rigid or flexible - can be used to contain the drug.

All references, including publications, patent applications, and patents, cited herein are incorporated by reference in their entirety and to the same extent as if each reference were individually and specifically indicated to be incorporated by reference and were set forth in its entirety herein.

All headings and sub-headings are used herein for convenience only and should not be constructed as limiting the invention in any way.

The use of any and all examples, or exemplary language (e.g. such as) provided herein, is intended merely to better illuminate the invention and does not pose a limitation on the scope of the invention unless otherwise claimed. No language in the specification should be construed as indicating any non-claimed element as essential to the practice of the invention. The citation and incorporation of patent documents herein is done for convenience only and does not reflect any view of the validity, patentability, and/or enforceability of such patent documents.

This invention includes all modifications and equivalents of the subject matter recited in the claims appended hereto as permitted by applicable law.

### BRIEF DESCRIPTION OF THE DRAWINGS:

The invention will be explained more fully below in connection with a preferred embodiment and with reference to the drawings in which:
- Figure 1: show a pen needle unit for an insulin pen system.
- Figure 2: show the method for bending the back needle part of the needle cannula.
- Figure 3: show the method of figure 2 with the back needle bended.
- Figure 4: show the pen needle container according to the present invention engaged in the method of bending the front needle part of the needle cannula.
- Figure 5: show the pen needle container locked to the pen needle assembly after use.

The figures are schematic and simplified for clarity, and they just show details, which are essential to the understanding of the invention, while other details are left out. Throughout, the same reference numerals are used for identical or corresponding parts.

### DETAILED DESCRIPTION OF EMBODIMENT:

When in the following terms as "upper" and "lower", "right" and "left", "horizontal" and "vertical", "clockwise" and "counter clockwise" or similar relative expressions are used, these only refer to the appended figures and not to an actual situation of use. The shown figures are schematic representations for which reason the configuration of the different structures as well as there relative dimensions are intended to serve illustrative purposes only.

In that context it may be convenient to define that the term "distal end" in the appended figures is meant to refer to the end of the needle cannula penetrating the patient whereas the term "proximal end" is meant to refer to the opposite end pointing away from the patient in a situation of use.

Figure 1 discloses a prior art pen needle unit as known from ISO 11608. A needle cannula 6 is secured to a hub 3 e.g. by gluing the needle cannula 6 and the hub 3 together using glue 7, however other fastening means could be used. The pen needle assembly is delivered to the user in a container 4 which is sealed at the open end with a peel foil 1. The needle cannula has an injection part or front part with a length /1 which enters into the skin of the user during injection and an opposite cartridge part or back part with a length /2 which enters into the cartridge containing the drug to be injected. The injection part is usually covered by an inner cap 5 and the interior part of the container 4 is usually sterilized.

Figure 2 discloses two identical needle containers 10, 20. The first needle container 10 supports a first needle assembly 11 and the second needle container 20 supports a second needle assembly 21. The first needle container 10 has a first proximal open end 12 and a first distal closed end 13. The second needle container 20 has a second proximal end 22 and a second distal closed end 23.

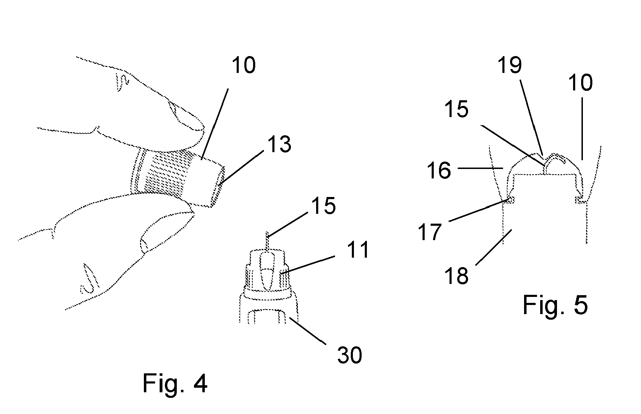

When the first pen needle assembly 11 has been used, the user can choose to use the first closed distal end 13 of the first needle container 10 to bend the front part 15 of the needle cannula 6 as explained later with reference to figure 4. Once the user has bended the front part 15 (if the user so chooses) and the user has returned the first needle assembly 11 to the first needle container 10 then the user inserts the second distal closed end 23 of the second pen needle container 20 into the first proximal open end 12 of the first container 10 and squeezes the first needle container 10 and the second pen needle container 20 towards each other as illustrated by the arrow "A", thereby using the second distal closed end 23 of second needle container 20 to bend the first back needle part 14 of the first needle cannula 6.

Figure 3 show the second distal closed end 23 of the second pen needle container 20 actually engaging and bending the first back needle part 14. The distal closed end 13, 23 of each needle container 10, 20 has a conical shape to better deflect the back needle part 14 of the needle cannula 6.

Figure 4 discloses the first needle assembly 11 mounted on an injection device 30. When the user has finalized an injection and prior to removing the first needle assembly 11 from the injection device 30, the user can utilize the first distal closed end 13 of the first needle container 10 to bend and thereby immobilize the front part 15 of the needle cannula 6 of the first needle assembly 11.

The injection device 30 can be any kind of injection device for injecting a drug into the body of a patient. Such injection devices are usually provided with tactile means informing the patient on which type of drug is contained in the injection device. However, other means can be provided to differentiate the injection device. The injection devices could e.g. be provided with a mechanical or electronic sound generator which provides a distinct sound depending on the type of drug contained in the injection devices. The sound generator could e.g. be incorporated in the dose setting mechanism such that the injection devices provide different click-sounds depending on the drug in the injection devices.

As further disclosed in figure 5, the first distal closed end 13 of the first needle container 10 can be provided with a plurality of arms 16 carrying inwardly pointing protrusions 17 at the distal end. These arms 16 and protrusions 17 are shaped to engage and lock to openings or indentations formed in the hub of the first needle assembly 11. Further, the first distal closed end 13 can be provided with a dot-shaped protrusion 19 which deflects the tip of the needle cannula during the initial phase of bending.

When the needle container 10 is locked to the hub 18 as disclosed in figure 5, the user can insert a different needle container into open end of the hub 18 and bend the back needle 14 as previously explained.

Some preferred embodiments have been shown in the foregoing, but it should be stressed that the invention is not limited to these, but may be embodied in other ways within the subject matter defined in the following claims.

## Claims

1. A pen needle container (4, 10, 20) for supporting a pen needle assembly (11, 21) which pen needle assembly (11, 21) comprises a needle cannula (6) mounted in a hub (3) such that a front needle part (15) of the needle cannula (6) extend in distal direction from the hub (3) and a back needle part (14) of the needle cannula (6) extend in proximal direction from the hub (3), and wherein,
the needle container (4, 10, 20) supports the pen needle assembly (11, 21) by engaging the hub (3) of the pen needle assembly (11, 21), the pen needle container (4, 10, 20) having a proximal open end (12, 22) and a distal closed end (13, 23), and wherein,
the distal closed end (13, 23) is shaped to engage and bend the front needle part (15) and/or the back needle part (14) of the needle cannula (6) of the pen needle assembly (11,21) upon impact.

2. A pen needle container (4, 10, 20) according to claim 1, wherein, the distal closed end (13, 23) has a conical or sphere shape.

3. A pen needle container (4, 10, 20) according to claim 1, wherein, the distal closed end (13, 23) has a protrusion (19).

4. A pen needle container (4, 10, 20) according to claim 1 or 2, wherein, the closed distal end (13, 23) is provided with a track for guiding the needle part (14, 15) during bending.

5. A pen needle container (4, 10, 20) according to previous claims, wherein, the distal closed end (13, 23) is harder than the remaining part of the pen needle container (4, 10, 20).

6. A pen needle container (4, 10, 20) according any of the previous claims, wherein, the distal closed end (13, 23) carries an insert.

7. A pen needle container (4, 10, 20) according any of the previous claims, wherein the distal closed end (13, 23) is provided with locking means for locking the pen needle container (4, 10, 20) to the pen needle assembly (11, 21).

8. A pen needle container (4, 10, 20) according any of the previous claims, wherein the locking means is a plurality of arms (16) where one or more of arms (16) carries a inwardly pointing protrusion (17) at the distal end of the arm (16).

9. A method for bending the back needle part (14) of a pen needle assembly (11, 21), which method comprises the steps of:
(i) providing a first pen needle container (10) containing a used pen needle assembly (11),
(ii) providing a second pen needle container (20),
(iii) entering the closed distal end (23) of the second pen needle container (20) into the proximal open end (12) of the first pen needle container (10),
(iv) pressing the first pen needle container (10) and the second pen needle container (20) towards each other.

10. A method for bending the front needle part (15) of a pen needle assembly (11, 21), which method comprises the steps of:
(i) providing a pen needle assembly (11, 21) having a front needle part (15) of the needle cannula (6),
(ii) providing a pen needle container (4, 10, 20),
(iii) pressing the closed distal end (13, 23) of the pen needle container (4, 10, 20) and the front needle part (15) of the needle cannula (6) against each other.
